# EUROPEAN PATENT APPLICATION

(11) **EP 4 461 209 A1**
(43) Date of publication of application: **13.11.2024**
(21) Application number: 23737077.0
(22) Date of filing: 05.01.2023
(51) Int. Cl.: A61B 5/00, A61N 1/36, A61N 5/06, A61N 5/00, A61B 18/18

(54) **BEAUTY DEVICE CONTROL SYSTEM BASED ON OPTICAL ASSEMBLY, AND BEAUTY DEVICE**

(30) Priority: 07.01.2022 CN 202210017620
(71) Applicant: Xiamen Solex High-Tech Industries Co., Ltd., Xiamen, Fujian 361028 (CN)
(72) Inventor: JIN, Chenying, Xiamen, Fujian 361028 (CN); LIU, Tingke, Xiamen, Fujian 361028 (CN); WEI, Fan, Xiamen, Fujian 361028 (CN); LIN, Caigui, Xiamen, Fujian 361028 (CN)
(74) Representative: Verscht, Thomas Kurt Albert
(86) International application number: PCT/CN2023/070579
(87) International publication number: WO 2023/131214

(57) **Abstract**

A beauty device control system based on an optical assembly (10), and a beauty device. The beauty device control system comprises: an optical assembly (10) and a control module (20), the optical assembly (10) being used for acquiring a skin image when a beauty device works, and analyzing movement data of the beauty device on the basis of the skin image, and the control module (20) being connected to the optical assembly (10) so as to control, on the basis of the movement data, output gears and/or working modes of the beauty device. The optical assembly (10) measures the movement data of the beauty device, and the control module (20) automatically adjusts the output gears and/or the working modes, so that the user experience can be improved.

## Description

### FIELD OF THE DISCLOSURE

The present disclosure relates to the technique field of personal care products, and in particular is a control system of a beauty device based on an optical assembly and the beauty device.

### BACKGROUND OF THE DISCLOSURE

With the progress and development of society, beauty pursuit of person is getting higher and higher, various beauty care devices are also emerged accordingly in view of love of beauty from person and widely accepted by person. With a rapid popularization of the beauty devices, requirements of person for the beauty devices are also higher and higher, the conventional beauty devices can merely output by choosing a certain fixed gear position and cannot be automatically adjusted according to a way of use, which is not smart enough. Therefore, when beauty care is performed at a single point, intensity is too large, when repeated movement operation is performed at a section, an application area is too large, resulting in intensity of the body feeling being too small, that is, a moving state cannot be recognized so as to be automatically adjusted to ensure a good experience.

In addition, beauty care of products of the beauty devices on the market selects a designated gear position basically through the button, and output cannot be automatically adjusted according to the way of use, which is not smart enough. With respect to a small part of the beauty devices, multi-axis acceleration or a gyroscope sensors (3-axis, 6-axis, etc.) are used to identify a movement (an acceleration sensor identifies variations in acceleration, a gyroscope sensor identifies variations in inclination), however, when it moves at a fixed inclination angle at a constant speed during use, a current movement state cannot be identified by the acceleration sensor.

### BRIEF SUMMARY OF THE DISCLOSURE

The main objective of the present disclosure is to provide a control system of a beauty device based on an optical assembly and the beauty device, which overcomes the deficiencies of the beauty device in the existing techniques, detects movement data of the beauty device using the optical assembly, automatically adjusts an output gear position and/or a working mode through a control module, and improves the user experience.

A technical solution of the present disclosure is as follows:
On the one hand, a control system of a beauty device based on an optical assembly, it comprises: the optical assembly and a control module; the optical assembly is used to obtain skin images when the beauty device is working, and obtain movement data of the beauty device based on the skin images by analyzing; the control module is connected to the optical assembly for controlling an output gear position and/or a working mode of the beauty device based on the movement data.

Preferably, the optical assembly comprises a lens, a light-emitting diode, an imager and an image analyzing chip; a working head of the beauty device comprises a window; the lens is disposed on the window; the light-emitting diode, the imager, and the image analyzing chip are respectively disposed on a rear side of the lens; light emitted by the light-emitting diode illuminates a skin surface through the lens, light reflected from the skin surface is transmitted to the imager, and the imager generates the skin images; the image analyzing chip is connected to the imager to receive the skin images to be converted into the movement data of the beauty device; the control module is connected to the image analyzing chip to control the output gear position and/or the working mode of the beauty device based on the movement data.

Preferably, the optical assembly further comprises a PCB board; the light-emitting diode, the imager and the image analyzing chip are disposed on the PCB board.

Preferably, the PCB board comprises an opening; the imager is disposed on a side of the PCB board away from the lens and receives the light reflected from the skin surface through the opening; the light-emitting diode is disposed on a side of the PCB board adjacent to the lens.

Preferably, the light-emitting diode and the imager are disposed on a same side of the PCB board.

Preferably, the imager and the image analyzing chip are disposed on a same side of the PCB board.

Preferably, the light-emitting diode is disposed on a side surface of the lens; and the imager directly faces the lens.

Preferably, the imager and image analyzing chip are integrally disposed on a chip of an optical sensor; or; the imager and image analyzing chip are independently disposed.

Preferably, the optical assembly obtains the skin images when the beauty device is working and obtains the movement data of the beauty device based on the skin images by analyzing, specifically comprising:
The optical assembly continuously captures the skin images when the beauty device is working;
A moving direction and a moving distance of the beauty device is determined by analyzing based on variations of feature point positions of the various skin images.

Preferably, the control module is connected to the optical assembly to control the output gear position of the beauty device based on the movement data, specifically comprising:
The control module receives the movement data; the movement data comprises a moving distance within a preset time of the beauty device;
A moving speed of the beauty device is obtained based on the moving distance within the preset time;
The output gear position of the beauty device is adjusted based on the moving speed of the beauty device.

Preferably, the control module is connected to the optical assembly to control the working mode of the beauty device based on the movement data, specifically comprising:
The control module receives the movement data; the movement data comprises a moving direction and a moving distance within a preset time of the beauty device;
A moving path of the beauty device is obtained based on the moving direction and the moving distance within the preset time;
The working mode of the beauty device is adjusted based on the moving path of the beauty device.

On the other hand, a beauty device, it comprises a handheld body and a working head; it further comprises the control system of the beauty device based on the optical assembly; and the optical assembly is disposed in the working head.

Preferably, the beauty device comprises a facial beauty device.

Preferably, the beauty device comprises a hair removal device.

Compared with the existing techniques, the present disclosure has the following advantages:
(1) The working head of the beauty device of the present disclosure comprises the window, the optical lens is disposed on the window, the light-emitting diode is disposed on the side surface of the optical lens, and the imager and the image analyzing chip are disposed on the rear side of the optical lens; when the working head is used while fitting the skin, the light emitted by the light-emitting diode is transmitted to the window and illuminates the skin surface through the optical lens, the light reflected from the skin surface is transmitted to the micro imager through the optical lens, the micro imager continuously captures images at given time intervals, different images generated in the moving process are transmitted to the image analyzing chip for digitalization, the image analyzing chip outputs the moving direction and the moving distance of the working head to the control module, the control module obtains the moving speed and the moving path based on the moving direction and the moving distance and automatically control the output gear position and/or the working mode of the beauty device, so as to improve the user experience;
(2) The optical assembly of the present disclosure comprising the lens, the light-emitting diode, the imager and the image analyzing chip has a simple structure and a low weight, which does not lead to increasing in a volume or a weight of the beauty device; furthermore, the light-emitting diode, the imager and the image analyzing chip can be integrally disposed in the inherent PCB board of the beauty device, which does not lead to needs for a redundant PCB board, and costs are saved;
(3) The control module of the present disclosure can detect the moving speed and/or the moving path of the beauty device (the working head) based on the movement data output by the image analyzing chip, and the output gear position is automatically adjusted according to the moving speed, so as to obtain same or similar body feeling when beauty operation is performed at a single point, when the beauty operation is performed in a rapid movement, and when the beauty operation is performed in a section; furthermore, with respect to the beauty device having multiple working modes, the working modes can also be automatically adjusted according to the moving path to meet diversified needs of the user.

The foregoing description is merely a summary of the technical solution of the present disclosure, and specific embodiments of the present disclosure are illustrated below in order to enable the technical methods of the present disclosure to be more clearly understood so as to be implemented in accordance with the specification and to enable the aforementioned and other objectives, features and advantages of the present disclosure to be more apparent and understandable.

The specific embodiments of the present disclosure are specifically described in combination with the following description and the accompanying drawings, and the aforementioned and other objectives, the advantages and the features of the present disclosure will be more apparent to technical persons skilled in the art.

### BRIEF DESCRIPTION OF THE DRAWINGS

FIG. 1 shows a structural diagram of a control system of Embodiment 1 of the present disclosure;
FIG. 2 shows a sectional view of an optical assembly of the control system of Embodiment 1 of the present disclosure;
FIG. 3 shows a perspective view of the optical assembly of the control system of Embodiment 1 of the present disclosure;
FIG. 4 shows a structural diagram of a control system for a facial beauty device of Embodiment 2 of the present disclosure;
FIG. 5 shows a diagrammatic view of an overall structure of the facial beauty device of Embodiment 2 of the present disclosure;
FIG. 6 shows a sectional view of the facial beauty device of Embodiment 2 of the present disclosure;
FIG. 7 shows an exploded perspective view of the facial beauty device of Embodiment 2 of the present disclosure;
FIG. 8 shows an exploded perspective view of main components within a working head of the facial beauty device of Embodiment 2 of the present disclosure;
FIG. 9 shows a circuit diagram of the control system of the facial beauty device of Embodiment 2 of the present disclosure; wherein (a) shows a circuit diagram of an input of an optical sensor; (b) shows a circuit diagram of an MCU controller; and (c) shows a circuit diagram of an output of a radio frequency;
FIG. 10 shows a diagrammatic view of an overall structure of a hair removal device of Embodiment 3 of the present disclosure.

### DETAILED DESCRIPTION OF THE EMBODIMENTS

The technical solutions in the embodiments of the present disclosure will be clearly and completely described in combination with the accompanying drawings in the embodiments of the present disclosure; it is obvious that the described embodiments are some embodiments of the present disclosure instead of all embodiments, all other embodiments fall within the protection scope of the present disclosure provided that they are obtained by ordinary technical persons skilled in the art based on the embodiments of the present disclosure without creative works.

In the description of the present disclosure, it should be noted that the terms "including", "comprising" or any other variations thereof are intended to cover non-exclusive inclusion, so that a process, a method, a product or a device including a series of elements not only comprises these elements, but also comprises other elements not expressly listed, or comprises elements inherent to the process, the method, the product or the device. Without further limitation, an element limited by a sentence "comprising a ......" does not exclude an existence of another identical element in the process, the method, the product or the device comprising the element.

In the description of the present disclosure, it should be noted that the terms, such as "upper", "lower", "inner", "outer", "top/bottom", indicate orientations or positional relationships based on orientations and positional relationships shown in the drawings, so as to easily describe the present disclosure and simplify the description, rather than indicating or implying that the referenced or implied device or element should have a specific orientation or be constructed and operated in a specific orientation and therefore should not be understood as a limitation of the present disclosure. Further, the terms "first" and "second" are merely used for description and can not to be understood as indicating or implying relative importance.

In the description of the present disclosure, unless otherwise specified and limited, it should be noted that the terms, such as "installed", "disposed", "sleeved/socketed", "connected", should develop broad understanding, for example, "connected" can be a fixed connection, a detachable connection, or an integrated connection, a mechanical connection, an electrical connection, a direct connection, an indirect connection through an intermediator, or communication between inner portions of two members, for ordinary technical person skilled in the art, specific meaning of the terms in the present disclosure can be understood under specific conditions.

### Embodiment 1

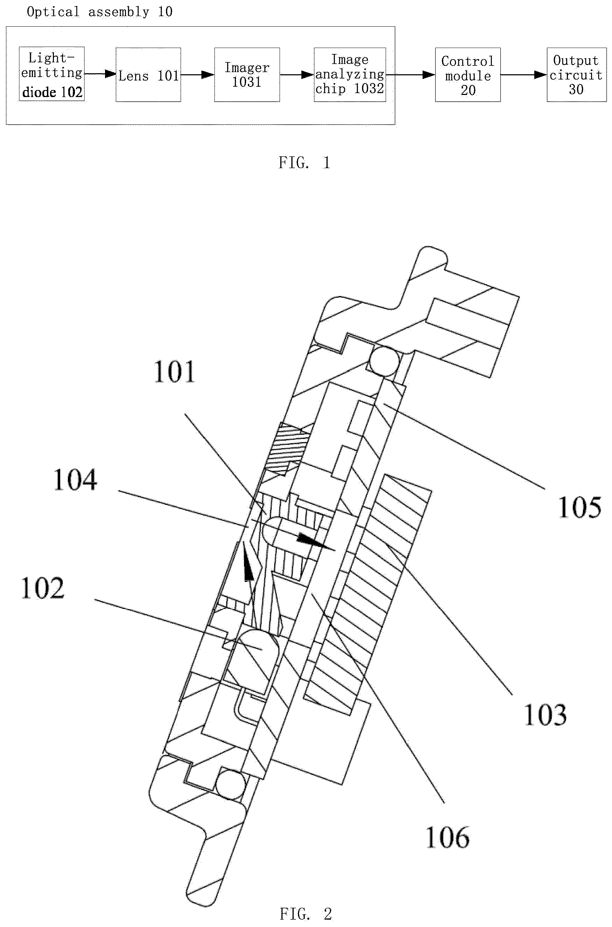

Referring to FIGS. 1-3, a control system of a beauty device based on an optical assembly comprises: the optical assembly 10 and a control module 20; the optical assembly 10 comprises a lens 101, a light-emitting diode 102, an imager 1031 and an image analyzing chip 1032; a working head of the beauty device comprises a window 104; the lens 101 is disposed on the window 104; the light-emitting diode 102, the imager 1031, and the image analyzing chip 1032 are respectively disposed on a rear side of the lens 101; light emitted by the light-emitting diode 102 illuminates a skin surface through the lens 101, light reflected from the skin surface is transmitted to the imager 1031, and the imager 1031 generates skin images; the image analyzing chip 1032 is connected to the imager 1031 to receive the skin images to be converted into movement data of the beauty device; the control module 20 is connected to the image analyzing chip 1032 to control an output circuit 30 to adjust output gear positions and/or working modes based on the received movement data.

In this embodiment, the lens 101 is an optical lens 101. The light-emitting diode 102 can be an ordinary light-emitting diode or a light-emitting diode patch. The imager 1031 is a micro imager. The image analyzing chip 1032 is a DSP (digital microprocessor) chip. The control module 20 comprises an MCU controller, and the MCU controller is disposed on a main circuit board 23.

When the working head of the beauty device of this embodiment is used while fitting a skin, the light emitted by the light-emitting diode 102 illuminates the skin surface through the lens 101, the light reflected from the skin surface returns to the imager 1031 through the lens 101, the imager 1031 continuously captures images at given time intervals and transmits different images generated in a moving process to the image analyzing chip 1032 for digitalization, so as to determine the movement data of the working head (e.g., a moving direction and a moving distance). The image analyzing chip 1032 transmits the movement data of the working head on the skin to the control module 20, and the MCU obtains a moving speed and/or a moving path of the working head on the skin according to the movement data by calculating and controls the output gear positions and/or the working modes of the beauty device according to the moving speed and/or the movement path.

In this embodiment, the optical assembly 10 further comprises a PCB board 105; the light-emitting diode 102, the imager 1031 and the image analyzing chip 1032 are disposed on the PCB board 105.

In one embodiment, in order to reduce a volume of the PCB board 105, the imager 1031 and the light-emitting diode 102 are disposed on two sides of the PCB board 105, the imager 1031 is disposed on a side of the PCB board away from the lens 101, and the light-emitting diode 102 is disposed on a side of the PCB board 105 adjacent to the lens 101. The PCB board 105 comprises an opening 106 so as to enable the imager 1031 to receive reflected light, and the imager 1031 receives the light reflected from the skin surface through the opening 106.

In another embodiment, when the PCB board 105 comprises a sufficient space for setting the light-emitting diode 102 and the imager 1031 on a same side, both the light-emitting diode 102 and the imager 1031 can be disposed on the side of the PCB board 105 adjacent to the lens 101.

In order to facilitate the imager 1031 to be electrically connected to the image analyzing chip 1032, the imager 1031 and the image analyzing chip 1032 are disposed on a same side of the PCB board 105.

In this embodiment, in order to enable the light emitted by the light-emitting diode 102 and passing through the lens 101 to be not coincide with the light reflected from the skin surface, the light-emitting diode 102 is disposed on a side surface of the lens 101, while the imager 1031 directly faces the lens 101. In one embodiment, incoming light projected onto the lens 101 by the light-emitting diode 102 can define an angle of 45 degrees, i.e., the light-emitting diode 102 and the lens 101 define 45 degrees.

In one embodiment, the imager 1031 and the image analyzing chip 1032 are integrally disposed on a chip of an optical sensor, i.e., functions of the imager 1031 and the image analyzing chip 1032 are realized by the optical sensor so as to further save an occupied area of the PCB board 105. In another embodiment, the imager 1031 and the image analyzing chip 1032 can also be independently disposed for easy replacement in case one of the imager 1031 and the image analyzing chip 1032 fails.

In this embodiment, the image analyzing chip 1032 is connected to the imager 1031 to receive the skin images to be converted into movement data of the beauty device, specifically comprising:
The image analyzing chip 1032 receives the skin images continuously captured by the imager 1031;
The moving direction and the moving distance of the beauty device are determined by analyzing based on variations of feature point positions on the various skin images.

The control module 20 is connected to the image analyzing chip 1032 to control the output gear position of the beauty device based on the movement data, specifically comprising:
The control module 20 receives the movement data; the movement data comprises the moving distance within a preset time of the beauty device;
A moving speed of the beauty device is obtained based on the moving distance within the preset time;
The output gear position of the beauty device is adjusted based on the moving speed of the beauty device.

Specifically, when the control module 20 recognizes that the moving speed of the beauty device increases and is greater than a preset value or lasts a preset time, which indicates that the beauty device is moving rapidly, the output gear positions of the beauty device can be automatically adjusted to a higher gear position, and no further adjustment is performed when the highest gear position is reached; when the control module 20 recognizes that the moving speed of the beauty device slows down and is less than the preset value or lasts the preset time, which indicates that the beauty device is decelerated, the output gear positions of the beauty device can be automatically adjusted to a lower gear position; when the control module 20 recognizes that the moving speed of the beauty device is 0 and lasts the preset time, the gear positions can be automatically adjusted to the lowest gear position, further, when it recognizes that the moving speed of the beauty device is 0 and lasts another preset time more than the preset time, in order to prevent a motor from burning out due to blocking of the working head 1, a power supply can be automatically shut down and prompted.

With respect to the beauty device having multiple working modes, the control module 20 is connected to the image analyzing chip 1032 to control the working modes of the beauty device based on the movement data, specifically comprising:
The control module 20 receives the movement data; the movement data comprises the moving direction and the moving distance within the preset time of the beauty device;
The moving path of the beauty device is obtained based on the moving direction and the moving distance within the preset time;
The working modes of the beauty device are adjusted based on the moving path of the beauty device.

An example for obtaining the moving path is as follows:
If a ratio of the moving distance in an X-axis and the moving distance in a Y-axis shows a linear variation with a relatively fixed slope, the moving path is judged to be a straight movement; if the moving distance in the X-axis and the moving distance in the Y-axis define alternate increasing and decreasing variations, the moving path is judged to be a circular movement.

Therefore, the working head the beauty device of this embodiment comprises the window 104, the optical lens 101 is disposed on the window 104, the light-emitting diode 102 is disposed on the side surface of the optical lens 101, and the imager 1031 and the image analyzing chip 1032 are disposed on the rear side of the optical lens 101; when the working head is use while fitting a skin, the light emitted by the light-emitting diode 102 is transmited to the window 104 by the optical lens 101 and illuminates the skin surface, the light reflected from the skin surface is transmitted the micro imager 1031 through the optical lens 101, the micro imager 1031 continuously captures the images at the given time intervals, different images generated in the moving process are transmitted to the image analyzing chip 1032 for digitalization, the image analyzing chip 1032 outputs the moving direction and the moving distance of the working head to the control module, the control module 20 obtains the moving speed and the moving path based on the moving direction and the moving distance and automatically adjusts the output gear positions according to the moving speed, so as to obtain same or similar body feelings when beauty care is performed at a single point, when the beauty care is performed in a rapid movement, and when the beauty care is performed in a section; furthermore, with respect to the beauty device having the multiple working modes, the working modes can be automatically adjusted according to the moving path to meet diversified needs of the user.

### Embodiment 2

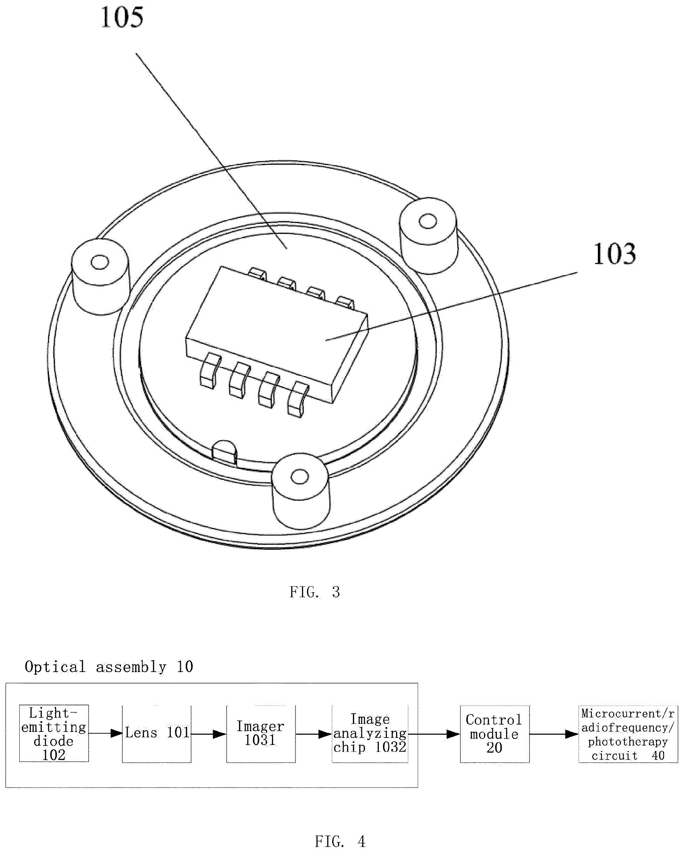

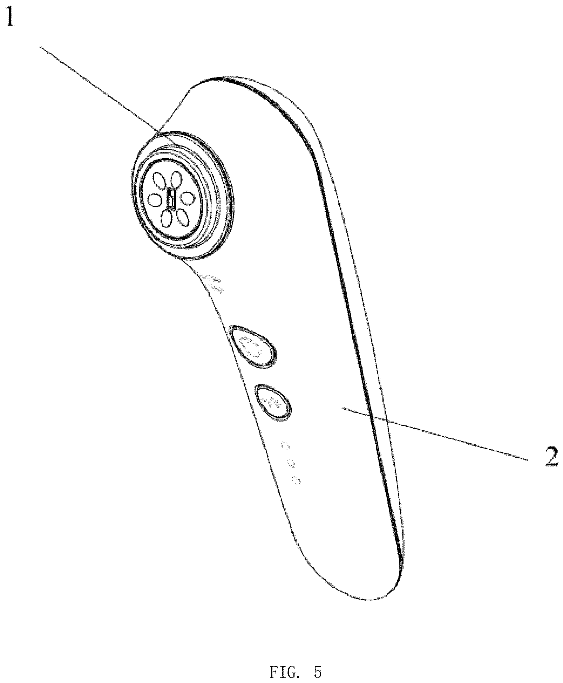

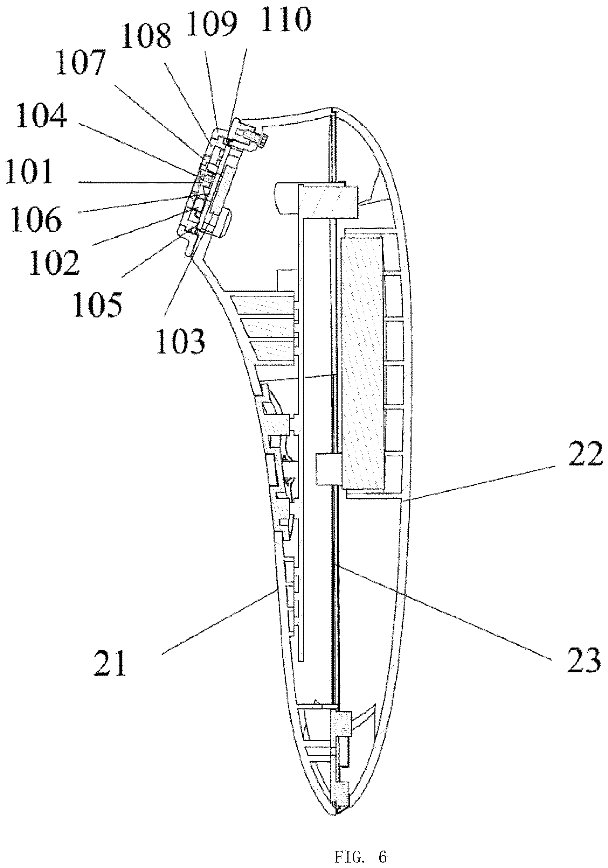

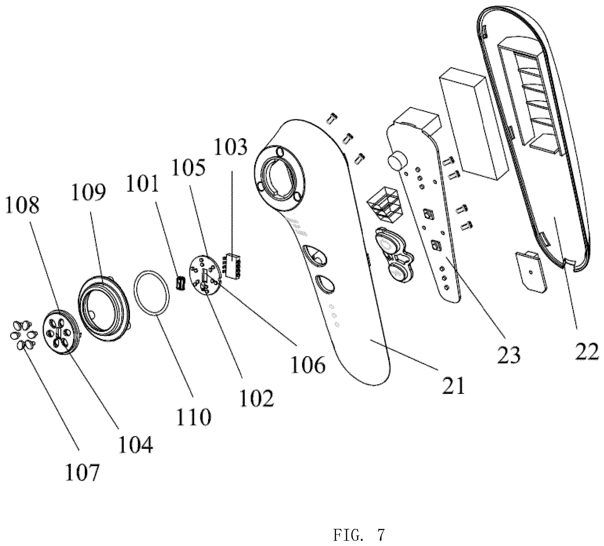

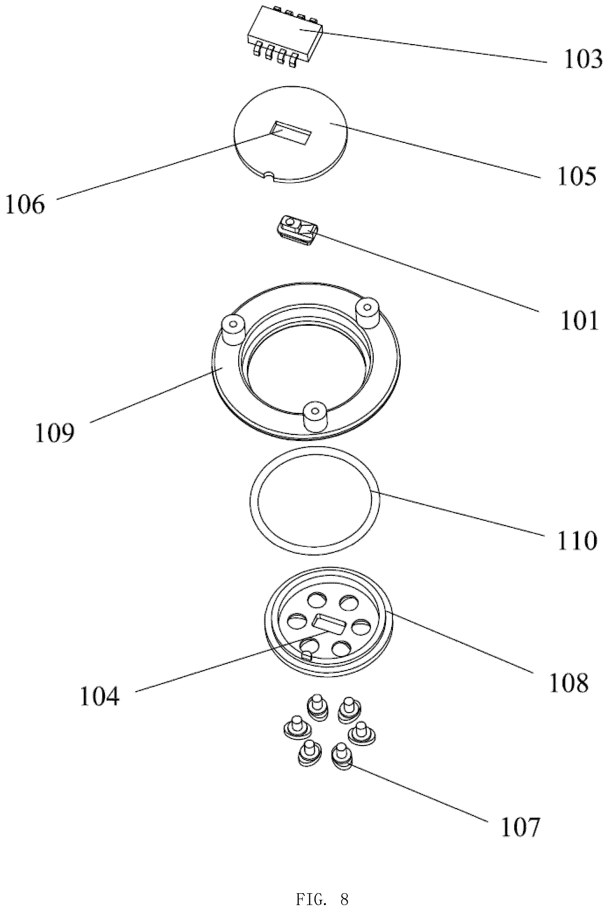

Referring to FIGS. 4-8, a facial beauty device of this embodiment comprises a handheld body 2 and a working head 1; and it further comprises a control system of a facial beauty device based on an optical assembly.

The control system of the facial beauty device based on the optical assembly comprises the optical assembly 10 and a control module 20. The optical assembly 10 comprises a lens 101, a light-emitting diode 102, an imager 1031 and an image analyzing chip 1032; the working head of the facial beauty device comprises a window 104; the lens 101 is disposed on the window 104; the light-emitting diode 102, the imager 1031, and the image analyzing chip 1032 are respectively disposed a rear side of the lens 101; light emitted by the light-emitting diode 102 illuminates a skin surface through the lens 101, and light reflected from the skin surface is transmitted to the imager 1031, and the imager 1031 generates skin images; the image analyzing chip 1032 is connected to the imager 1031 to receive the skin images to be converted into movement data of the facial beauty device; the control module 20 is connected to the image analyzing chip 1032 to control a microcurrent/radiofrequency/phototherapy circuit 40 of the beauty device to adjust output gear positions and/or working modes based on the received movement data.

The optical assembly 10 in this embodiment is disposed in the working head, and the control module 20 is disposed in the handheld body.

The facial beauty device of this embodiment integrates beauty care functions of microcurrent, phototherapy and RF radiofrequency. It should be noted that in other embodiments, the facial beauty device can merely comprise one of the beauty care functions of the microcurrent, the phototherapy and the RF radiofrequency, or comprise other beauty care functions, and the present disclosure is specifically limited.

Specifically, the handheld body of this embodiment comprises an upper housing 21 and a lower housing 22, and the control module 20 is disposed in a cavity consisting of the upper housing 21 and the lower housing 22. The working head of this embodiment comprises a fixed seat 109, a light-transmitting panel 108, a microcurrent/radiofrequency contact point 107, a microcurrent/radiofrequency/phototherapy PCB board 105 (the board on which the microcurrent/radiofrequency/phototherapy circuit 40 is disposed) and the optical assembly 10.

The light-transmitting panel 108 is connected to the microcurrent/radiofrequency/phototherapy PCB board 105 by passing through the fixed seat 109, and the light-transmitting panel 108 is fixedly connected to the fixed seat 109 by screws and the like. The microcurrent/radiofrequency/phototherapy PCB board 105 is connected to a main circuit board 23 (the main circuit board 23 comprises the control module 20, the control module 20 comprises an MCU controller). Radio frequency, microcurrent and phototherapy massages of the facial beauty device is achieved through a combination of the light-transmitting panel 108, the microcurrent/radiofrequency contact point 107, the microcurrent/radiofrequency/phototherapy PCB board 105 and so on are existing techniques, and a realization principle and an operation principle thereof are not specifically described in this embodiment.

In this embodiment, the window 104 is disposed on the light-transmitting panel 108. Specifically, the window 104 is disposed on a center of the light-transmitting panel 108.

In this embodiment, the light-emitting diode 102, the imager 1031 and the image analyzing chip 1032 are disposed on the microcurrent/radiofrequency/phototherapy PCB board 105.

In order to reduce a volume of the microcurrent/RF/phototherapy PCB board 105, the imager 1031 and the diode are diposed on two sides of the microcurrent/radiofrequency/phototherapy PCB board 105, the imager 1031 is disposed on a side of the microcurrent/radiofrequency/phototherapy PCB board 105 away from the lens 101, and the light-emitting diode 102 is disposed on a side of the microcurrent/radiofrequency/phototherapy PCB board 105 adjacent to the lens 101. The microcurrent/radiofrequency/phototherapy PCB board 105 comprises an opening 106 so as to enable the imager 1031 to receive reflected light, and the imager 1031 receives light reflected from the skin surface through the opening 106.

In order to further save an occupied area of the microcurrent/radiofrequency/phototherapy PCB board 105, the imager 1031 and image analyzing chip 1032 are integrally disposed on a chip 103 of an optical sensor, i.e. functions of the imager 1031 and the image analyzing chip 1032 are achieved by the chip 103 of the optical sensor.

In this embodiment, a waterproof gasket 110 is also disposed in the working head for waterproof, and a protection for internal parts of the working head 1 is achieved. Specifically, the waterproof gasket 110 can be tightly pressed against the fixed seat 109.

The specific implementation of the optical assembly 10 and the control module 20 of this embodiment is the same as Embodiment 1 and will not be repeated herein.

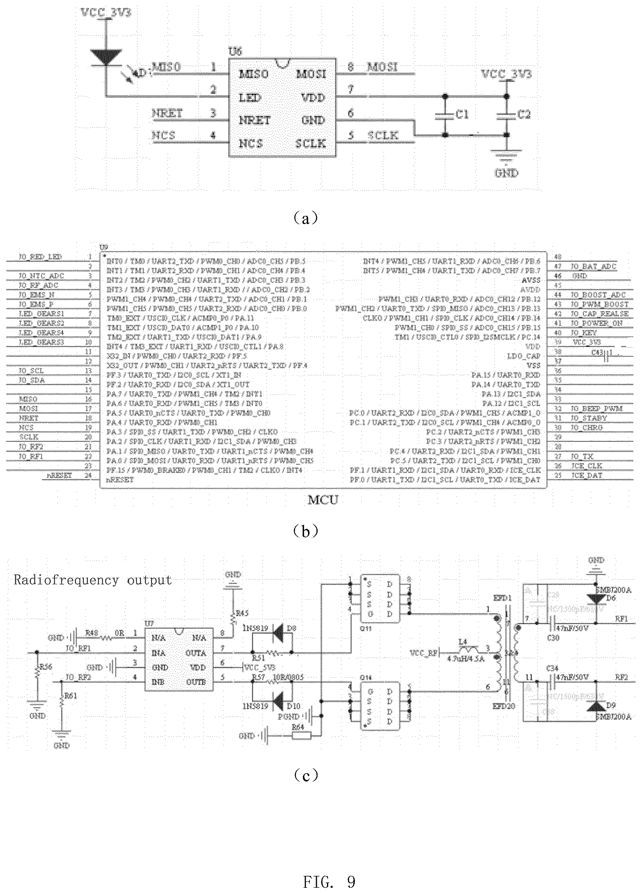

Referring to FIG. 9, a circuit diagram of the control system of the facial beauty device of this embodiment; wherein (a) shows a circuit diagram of an input of the optical sensor; (b) shows a circuit diagram of the MCU controller; and (c) shows a circuit diagram of an output of the radio frequency. Specifically, the optical sensor outputs the movement data of the facial beauty device (via MOSI interfaces) to the MCU controller, and the MCU controller outputs a radiofrequency signal (via IO_RF1/ IO_RF2 interfaces) to a radiofrequency module.

In this embodiment, when the control module 20 recognizes that the moving speed of the facial beauty device increases and is greater than a preset value or lasts a preset time, which indicates that the facial beauty device is moving rapidly, the output gear positions of the facial beauty device can be automatically adjusted to a higher gear position (specifically, a temperature of the radiofrequency increases, a magnitude of the microcurrent increases, an energy level of the phototherapy increases, etc.), and no further adjustment is performed when the highest gear position is reached; when the control module 20 recognizes that the moving speed of the facial beauty device slows down and is less than the preset value or lasts the preset time, which indicates that the facial beauty device is decelerated, the output gear positions of the facial beauty device can be automatically adjusted to a lower gear position (specifically, the temperature of the radiofrequency decreases, the magnitude of the microcurrent decreases, the energy level of the phototherapy decreases, etc.); when the control module 20 recognizes that the moving speed of the facial beauty device is 0 and lasts the preset time, the gear positions can be automatically adjusted to the lowest gear position, further, when it recognizes that the moving speed of the facial beauty device is 0 and lasts another preset time more than the preset time, in order to prevent a motor from burning out due to blocking of the working head 1, a power supply can be automatically shut down and prompted.

Specifically, a principle for adjusting a strength of the gear positions of the facial beauty device in a moving process is that different speed intervals correspond to strength values of different gear positions, specifically:
A lowest value of power output is set as Pmin, Pmin corresponds to a speed of Vmin; a highest value of the power output is set as Pmax, Pmax corresponds to a speed of Vmax; when a current detected moving speed of the facial beauty device is Vnow, a power corresponding to the current speed is Pv = Pmin + (Vnow* (Pmax-Pmin)) / (Vmax-Vmin). Variations in a power level correspond to different optical densities, microcurrent magnitudes, phototherapy energy levels, etc.

Further, with respect to the facial beauty device of this embodiment, if a ratio of the moving distance in an X-axis and the moving distance in a Y-axis shows a linear variation with a relatively fixed slope, the moving path is judged to be a straight movement; if the moving distance in the X-axis and the moving distance in the Y-axis define alternate increasing and decreasing variations, the moving path is judged to be a circular movement. In a straight reciprocating movement, simultaneous output are achieved by switching to the radiofrequency and red light to perform heating and massage in a wide range to accelerate blood circulation; in the circular movement in a small range, considering a range of use is mainly an eye corner, a mouth corner and other areas, a mode in which the radiofrequency and the microcurrent are alternately output is used to stimulate cellular activity and promote collagen fibers to contract.

It should be noted that if a variation of the moving path is detected in a fixed gear position, the working modes are adjusted, similarly, if a variation of the moving speed is detected in a certain working mode, the output gear positions are adjusted, i.e., the working modes and the output gear positions can be alternately adjusted.

Further, the image analyzing chip 1032 of the facial beauty device of this embodiment can also be used to pre-store facial feature images, such as fine wrinkles, roughness, etc., to increase judgment indicators.

### Embodiment 3

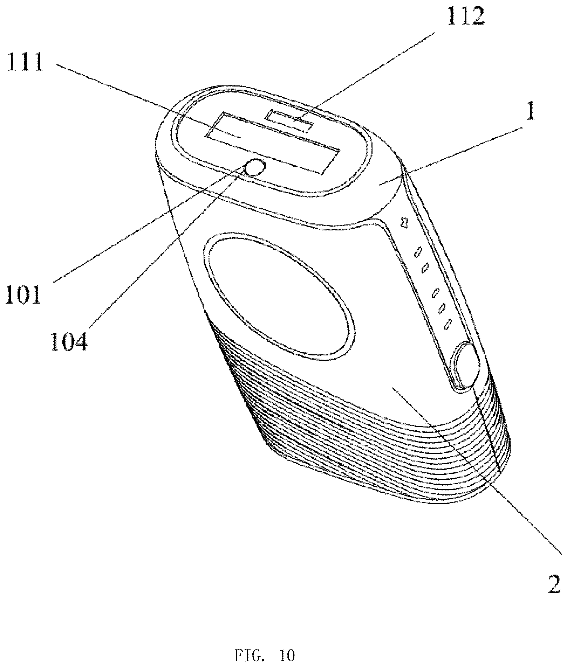

Referring to FIGS. 1-3 and 10, a hair removal device of this embodiment of comprises a handheld body 2 and a working head 1; and it also includes a control system of the hair removal device based on an optical assembly.

The control system of the hair removal device based on the optical assembly comprises the optical assembly 10 and a control module 20. The optical assembly 10 comprises a lens 101, a light-emitting diode 102, an imager 1031 and an image analyzing chip 1032; a working head of the hair removal device comprises a window 104; the lens 101 is disposed on the window 104; the light-emitting diode 102, the imager 1031, and the image analyzing chip 1032 are respectively disposed a rear side of the lens 101; light emitted by the light-emitting diode 102 illuminates a skin surface through the lens 101, light reflected from the skin surface is transmitted to the imager 1031, and the imager 1031 generates skin images; and the image analyzing chip 1032 is connected to the imager 1031 to receive the skin images to be converted into movement data of the hair removal device; the control module 20 is connected to the image analyzing chip 1032 to control an output circuit 30 of the hair removal device based on the received movement data to adjust output gear positions and/or working modes, specifically, light densities/energy levels of the hair removal device and the like are adjusted.

Components, positions thereof and connection relationships thereof of the control system of the hair removal device based on the ball and the optical assembly 10 are the same as the control system of the beauty device based on the optical assembly and are not described in this embodiment.

The optical assembly 10 in this embodiment is disposed in the working head, and the control module 20 is disposed in the handheld body.

The hair removal device of this embodiment comprises functions of hair removal and skin detection, etc. It should be noted that the hair removal device can merely have a hair removal function in other embodiments, and the present disclosure does not specifically limited.

Specifically, the working head of this embodiment comprises a light outlet 111, a skin detection point 112, the lens 101 and a corresponding window 104.

The specific implementation of the optical assembly 10 and the control module 20 of this embodiment is the same as Embodiment 1 and will not be repeated herein.

In this embodiment, when the control module 20 recognizes that the moving speed of the hair removal device increases and is greater than a preset value or lasts a preset time, which indicates that the hair removal device is moving rapidly, the output gear positions of the hair removal device can be automatically adjusted to a higher gear position (specifically, the optical densities/the energy levers, etc., of the hair removal device increase), and no further adjustment is performed when the highest gear position is reached; when the control module 20 recognizes that the moving speed of the hair removal device slows down and is less than the preset value or lasts the preset time, which indicates that the hair removal device is decelerated, the output gear positions of the hair removal device can be automatically adjusted to a lower gear position (specifically, the optical densities/the energy levers, etc., of the hair removal device decrease); when the control module 20 recognizes that the moving speed of the hair removal device is 0 and lasts the preset time, the gear positions can be automatically adjusted to the lowest gear position, further, when it recognizes that the moving speed of the hair removal device is 0 and lasts another preset time more than the preset time, in order to prevent a motor from burning out due to blocking of the working head 1, a power supply can be automatically shut down and prompted.

Specifically, a principle for adjusting a strength of the gear positions of the hair removal device in a moving process is that different speed intervals correspond to strength values of different gear positions, specifically:
A lowest value of power output is set as Pmin, Pmin corresponds to a speed of Vmin; a highest value of the power output is set as Pmax, Pmax corresponds to a speed of Vmax; when a current detected moving speed of the hair removal device is Vnow, a power corresponding to the current speed is Pv = Pmin + (Vnow* (Pmax-Pmin)) / (Vmax-Vmin). Variations in a power level correspond to different optical densities/energy levels, etc.

The aforementioned embodiments are merely preferred embodiments of the present disclosure, and the scope of the disclosure is not limited thereto. Thus, it is intended that the present disclosure cover modifications and variations provided they are made within the technical scope disclosed by the present disclosure based on the technical solutions and improved concepts of the present disclosure by any technical person skilled in the art.

### INDUSTRIAL APPLICABILITY

The present disclosure discloses a control system of a beauty device based on an optical assembly and the beauty device, the optical assembly is used to obtain the skin images when the beauty device is working and obtain the movement data of the beauty device based on the skin images by analyzing; the control module is connected to the optical assembly to control the output gear positions and/or the working modes of the beauty device based on the movement data. The present disclosure can be used in various beauty devices, the movement data of the beauty device is detected through the optical assembly, and the output gear positions and/or the working modes are automatically adjusted through the control module so as to improve the user experience.

## Claims

1. A control system of a beauty device based on an optical assembly, **characterized in that**, it comprises: the optical assembly and a control module; the optical assembly is used to obtain skin images when the beauty device is working, and obtain movement data of the beauty device based on the skin images by analyzing; the control module is connected to the optical assembly for controlling an output gear position and/or a working mode of the beauty device based on the movement data.

2. The control system of the beauty device based on the optical assembly according to claim 1, **characterized in that**, the optical assembly comprises a lens, a light-emitting diode, an imager and an image analyzing chip; a working head of the beauty device comprises a window; the lens is disposed on the window; the light-emitting diode, the imager, and the image analyzing chip are respectively disposed on a rear side of the lens; light emitted by the light-emitting diode illuminates a skin surface through the lens, light reflected from the skin surface is transmitted to the imager, and the imager generates the skin images; the image analyzing chip is connected to the imager to receive the skin images to be converted into the movement data of the beauty device; the control module is connected to the image analyzing chip to control the output gear position and/or the working mode of the beauty device based on the movement data.

3. The control system of the beauty device based on the optical assembly according to claim 2, **characterized in that**, the optical assembly further comprises a PCB board; the light-emitting diode, the imager and the image analyzing chip are disposed on the PCB board.

4. The control system of the beauty device based on the optical assembly according to claim 3, **characterized in that**, the PCB board comprises an opening; the imager is disposed on a side of the PCB board away from the lens and receives the light reflected from the skin surface through the opening; the light-emitting diode is disposed on a side of the PCB board adjacent to the lens.

5. The control system of the beauty device based on the optical assembly according to claim 3, **characterized in that**, the light-emitting diode and the imager are disposed on a same side of the PCB board.

6. The control system of the beauty device based on the optical assembly according to claim 3, **characterized in that**, the imager and the image analyzing chip are disposed on a same side of the PCB board.

7. The control system of the beauty device based on the optical assembly according to claim 2, **characterized in that**, the light-emitting diode is disposed on a side surface of the lens; and the imager directly faces the lens.

8. The control system of the beauty device based on the optical assembly according to claim 2, **characterized in that**, the imager and image analyzing chip are integrally disposed on a chip of an optical sensor; or; the imager and image analyzing chip are independently disposed.

9. The control system of the beauty device based on the optical assembly according to claim 1, **characterized in that**, the optical assembly obtains the skin images when the beauty device is working and obtains the movement data of the beauty device based on the skin images by analyzing, specifically comprising:
the optical assembly continuously captures the skin images when the beauty device is working;
a moving direction and a moving distance of the beauty device is determined by analyzing based on variations of feature point positions of the various skin images.

10. The control system of the beauty device based on the optical assembly according to claim 1, **characterized in that**, the control module is connected to the optical assembly to control the output gear position of the beauty device based on the movement data, specifically comprising:
the control module receives the movement data; the movement data comprises a moving distance within a preset time of the beauty device;
a moving speed of the beauty device is obtained based on the moving distance within the preset time;
the output gear position of the beauty device is adjusted based on the moving speed of the beauty device.

11. The control system of the beauty device based on the optical assembly according to claim 1, **characterized in that**, the control module is connected to the optical assembly to control the working mode of the beauty device based on the movement data, specifically comprising:
the control module receives the movement data; the movement data comprises a moving direction and a moving distance within a preset time of the beauty device;
a moving path of the beauty device is obtained based on the moving direction and the moving distance within the preset time;
the working mode of the beauty device is adjusted based on the moving path of the beauty device.

12. A beauty device, it comprises a handheld body and a working head; **characterized in that**: it further comprises the control system of the beauty device based on the optical assembly according to any one of claims 1-11; and the optical assembly is disposed in the working head.

13. The beauty device according to claim 12, **characterized in that**, the beauty device is a facial beauty device:
the working head comprises a fixed seat, a light-transmitting panel, a microcurrent/radiofrequency contact point, a microcurrent/radiofrequency/phototherapy PCB board; the imager and the image analyzing chip of the optical assembly are integrally disposed on a chip of an optical sensor;
the control module is connected to the optical assembly to control the working mode of the beauty device based on the movement data:
the control module receives the movement data; the movement data comprises a moving direction and a moving distance within a preset time of the beauty device;
a moving path of the beauty device is obtained based on the moving direction and the moving distance within the preset time; if a ratio of the moving distance in an X-axis and the moving distance in a Y-axis shows a linear variation with a relatively fixed slope, the moving path is judged to be a straight movement; if the moving distance in the X-axis and the moving distance in the Y-axis define alternate increasing and decreasing variations, the moving path is judged to be a circular movement;
the working mode of the beauty device is adjusted based on the moving path of the beauty device: in the straight reciprocating movement, simultaneous output are performed by switching to the radiofrequency and red light; in the circular movement in a small range, a mode in which the radiofrequency and the microcurrent are alternately output is used.

14. The beauty device according to claim 12, **characterized in that**, the beauty device comprises a hair removal device.
